# EUROPEAN PATENT SPECIFICATION

(11) **EP 2 289 409 B1**
(45) Date of publication and mention of the grant of the patent: **27.06.2018**
(21) Application number: 10174683.2
(22) Date of filing: 31.08.2010
(51) Int. Cl.: A61B 5/08, A61B 5/113, A61B 5/0205, G06F 19/00, A63B 24/00

(54) **Physiologic database and system for population modeling and method of population modeling**
Nichtinvasives Verfahren und System zur Überwachung physiologischer Merkmale
Procédé non invasif et système pour la surveillance des caractéristiques physiologiques

(30) Priority: 01.09.2009 US 275635 P; 26.08.2010 US 869586
(43) Date of publication of application: 02.03.2011
(73) Proprietor: adidas AG, 91072 Herzogenaurach (DE)
(72) Inventor: Derchak, P. Alexander, Oxnard, CA 93035 (US); Mitchnick, Mark, East Hampton, NY 11937 (US)
(74) Representative: Wegner, Hans

(56) References cited:
- US-A1- 2004 117 204
- US-A1- 2008 269 644
- US-B2- 6 858 006

## Description

### Field of the Invention

The present invention relates generally to methods and systems for processing physiological and athletic performance data. More particularly, the invention relates to a physiologic and/or athletic performance database and system for population modeling and a method of population modeling.

### Background Art

In medical diagnosis and treatment of a patient, it is often necessary to defer to physiological profiles of subjects that have presented similar physiological characteristics or symptoms to the patient. The physiological profiles are often created from various sensor signals representing physiological characteristics associated with the subjects.

Monitoring physiological and performance parameters of a subject can also be important in planning and evaluating athletic training and activity. A subject may exercise or otherwise engage in athletic activity for a variety of reasons, including, for example, maintaining or achieving a level of fitness, to prepare for or engage in competition, and for enjoyment. The subject may have a training program tailored to his or her fitness level and designed to help him or her progress toward a fitness or exercise goal. Physiological and performance parameters of a subject can provide useful information about the subject's progression in a training program, or about the athletic performance of the subject. In order to accurately appraise the subject's fitness level or progress toward a goal, it may be useful to determine, monitor, and record various physiological or performance parameters, and related contextual information.

Various methods and systems utilizing heart rate have been introduced to approximate effort and physiological stress during exercise. Convenient, practicable, and comfortable means of measuring pulmonary ventilation in non-laboratory conditions, however, have been scarce. While of good value, heart rate can only give an approximation as to the true physiological state of an athlete or medical patient, as it can be confounded by external factors including, for example, sleep levels, caffeine, depressants, beta blockers, stress levels, hydration status, temperature, etc. Furthermore, accurate use of heart rate to gauge physiological performance requires knowledge of the amount of blood flowing to the muscles, which in turn requires knowledge of the instantaneous stroke volume of the heart as well as the rate of pumping. These parameters can be difficult to determine while a subject is engaging in a physical activity.

As is well known in the art, there are a multitude of monitoring systems and associated methods available to acquire signals representing physiological and anatomical parameters. Illustrative are the systems and associated methods disclosed in U.S. Patent Nos. 6,840,907, issued January 11, 2005, 5,002,060, issued March 26, 1991, 6,790,183, issued September 14, 2004, 6,599,251, issued July 29, 2003, 6,454,719, issued September 24, 2002, 6,527,729, issued March 4, 2003, 6,015,388, issued January 18, 2000, and 6,047,203, issued April 4, 2000.

U.S. Patent No. 6,840,907 discloses a respiratory analysis system for monitoring a respiratory variable of a patient.

U.S. Patent No. 5,002,060 discloses a monitoring system adapted to simultaneously monitor cardiac and respiratory rates and characteristics and substantial changes in temperature of a subject.

U.S. Patent No. 6,790,183 discloses a lung sound diagnostic system for use in collecting, organizing, and analyzing lung sounds associated with the inspiration(s) and expiration(s) of a patient.

U.S. Patent No. 6,599,251 discloses non-invasive techniques for monitoring the blood pressure of a subject.

U.S. Patent No. 6,454,719 discloses techniques for determining the cardiac condition of a patient by a cardiac monitor apparatus using a respiration parameter, such as a current respiration signal or a respiration rate.

U.S. Patent No. 6,527,729 discloses a method for monitoring the progression of disease of a heart failure patient.

U.S. Patent No. 6,015,388 to Sackner et al. (VivoMetrics, Inc.) discloses a method for measuring respiratory drive, including determining a peak inspiratory flow and a peak inspiratory acceleration from a breath waveform derived from rib cage motion and abdominal motion using a plethysmograph or other external respiratory measuring device.

U.S. Patent No. 6,047,203 to Sackner et al. (VivoMetrics, Inc.) discloses physiological monitoring apparel worn by a monitored individual, the apparel having attached sensors for monitoring parameters reflecting pulmonary function, cardiac function, or the function of other organ systems. US2008/0269644 discloses a precision athletic aptitude and performance data analysis system.

Although the noted monitoring systems effectively acquire and transmit signals reflecting physiological and performance characteristics, the signals are limited in many respects. In many instances, the signals transmitted by the noted systems only reflect a few characteristics (e.g., respiratory rate, blood pressure, or temperature). Thus, to create an effective library of physiologic and/or performance data, multiple systems would need to be employed.

### BRIEF SUMMARY OF THE INVENTION

The present invention provides apparatuses and methods for creating a physiologic database or library that can be indexed to at least health, disease, patient characteristics, and acute medical crises or other dangerous conditions. The database can be structured and continuous/pseudo-continuous modeling statistics applied to provide novel insight into the natural history of disease and the response to treatment, and ultimately to improve care delivery.

The present invention also provides apparatuses and methods for creating an athletic performance database or library. The database can be structured and continuous/pseudo-continuous modeling statistics applied to provide novel insight into performance and progress of an athlete or group of athletes, as well as standing relative to other athletes.

The database (and information derivation approach), coupled with the wealth of relevant/real-world data provides improved insight into epidemiology, the natural history of various diseases, the impact of treatment, the true costs of diseases and treatments, and will ultimately improve patient medical care. The approach will also improve insight into performance and progress of an athlete or group of athletes, as well as standing relative to other athletes.

An embodiment of the present invention provides a database system for population modeling, the database system including a data acquisition subsystem configured to generate and transmit signals representing a characteristic the subject, and a processor subsystem in communication with the data acquisition subsystem and being configured to receive the signals and to transmit the signals to a database, the database being configured to receive and store the signals.

An embodiment of the present invention also provides a database for population modeling, the database including a storage unit configured to receive and store signals representing a characteristic of a subject, wherein the signals are received from a sensor, the sensor being positioned proximate to the subject's body, and being configured to generate and transmit the signals.

An embodiment of the present invention also provides a method for modeling a population, the method including receiving signals representing a characteristic of a subject, and storing the signals in a database in association with demographic information of the subject and performance history of the subject, wherein the database includes a storage unit.

### BRIEF DESCRIPTION OF THE DRAWINGS/FIGURES

Further features and advantages will become apparent from the following and more particular description of the preferred embodiments of the invention, as illustrated in the accompanying drawings, and in which like referenced characters generally refer to the same parts or elements throughout the views.
FIG. 1 is a schematic illustration of a physiology monitoring system, according to one embodiment of the invention.
FIG. 2 is a schematic illustration of a dual-paired electromagnetic coil arrangement, according to one embodiment of the invention.
FIG. 3 is a side view of a subject, showing the position of the dual-paired electromagnetic coil arrangement of Fig. 2 on the subject, according to one embodiment of the invention.
FIG. 4 is a perspective view of the subject, showing the position of electromagnetic coils on the front of the subject, according to one embodiment of the invention.
FIG. 5 is a plane view of the subject's back, showing the position of electromagnetic coils thereon, according to one embodiment of the invention.
FIGS. 6 and 7 are schematic illustrations of a multiple-paired electromagnetic coil arrangement, according to one embodiment of the invention.
FIG. 8 is a perspective view of a subject, showing the position of the multiple-paired electromagnetic coils shown in FIG. 6 on the front of the subject, according to one embodiment of the invention.
FIG. 9 is a plane view of the subject's back, showing the position of electromagnetic coils thereon, according to one embodiment of the invention.
FIGS. 10-12 are schematic illustrations of coil transmission axes provided by several multiple-paired coil embodiments of the invention.
FIG. 13 is a perspective view of a subject, showing alternative positions of the multiple-paired electromagnetic coils shown in FIG. 6 on the front of the subject, according to another embodiment of the invention.
FIG. 14 is a plane view of the subject's back, showing the positioning of three pairs of electromagnetic coils thereon, according to another embodiment of the invention.
FIG. 15 is a plane view of the subject's back, showing alternative positions of the paired electromagnetic coils shown in FIG. 14 thereon, according to another embodiment of the invention.
FIG. 16 is a perspective view of a subject, showing the position of six pairs of electromagnetic coils on the front and one side of the subject, according to another embodiment of the invention.
FIG. 17 is a plane view of the subject's back, showing the position of five pairs of electromagnetic coils on the back and both sides of the subject, according to another embodiment of the invention.

### DETAILED DESCRIPTION OF THE INVENTION

In the following any example and embodiment which does not fall under the scope of the independent claims is not part of the invention.

Unless defined otherwise, all technical and scientific terms used herein have the same meaning as commonly understood by one having ordinary skill in the art to which the invention pertains.

As used in this specification and the appended claims, the singular forms "a", "an", and "the" include plural referents unless the content clearly dictates otherwise.

The publications discussed herein are provided solely for their disclosure prior to the filing date of the present application. Nothing herein is to be construed as an admission that the present invention is not entitled to antedate such publication(s) by virtue of prior invention. Further, the dates of publication may be different from the actual publication dates, which may need to be independently confirmed.

### Definitions

The terms "respiratory parameter" and "respiratory characteristic", as used herein, mean and include a characteristic associated with the respiratory system and functioning thereof, including, without limitation, breathing frequency (fB), tidal volume (V_{T}), inspiration volume (V_{I}), expiration volume (V_{E}), minute ventilation (VE), inspiratory breathing time, expiratory breathing time, and flow rates (e.g., rates of change in the chest wall volume). The terms "respiratory parameter" and "respiratory characteristic" further mean and include inferences regarding ventilatory mechanics from synchronous or asynchronous movements of the chest wall compartments.

According to the present invention, flow rates and respiratory accelerations can be determined from a volume signal. Further, numerous inferences regarding ventilatory mechanics can be drawn from the degree of asynchrony in movement occurring amongst the discrete compartments that make up the chest wall.

The terms "respiratory system disorder", "respiratory disorder", and "adverse respiratory event", as used herein, mean and include any dysfunction of the respiratory system that impedes the normal respiration or ventilation process.

The terms "physiological parameter" and "physiological characteristic", as used herein, mean and include, without limitation, electrical activity of the heart, electrical activity of other muscles, electrical activity of the brain, pulse rate, blood pressure, blood oxygen saturation level, skin temperature, and core temperature.

The terms "spatial parameter" and "spatial characteristic", as used herein, mean and include a subject's orientation and/or movement.

The terms "patient" and "subject", as used herein, mean and include humans and animals.

Pulmonary ventilation, tidal volume, respiratory rate, and other associated respiratory characteristics can provide a reliable and practical measure of oxygen and carbon dioxide transpiration in a living body. Respiratory characteristics are directly related to exercise effort, physiological stress, and other physiological characteristics. One way to externally determine tidal volume is to measure the change in thoracic volume. Change in thoracic volume is caused by the expansion and contraction of the lungs. As the gas pressure in the lungs at the maxima and minima of the pressure ranges is equilibrated to surrounding air pressure, there is a very close and monotonic relationship between the volume of the lungs and the volume of air inspired.

Accurate measurement of the change in thoracic volume involves measuring the change in the diameter of the chest at the ribcage. Measurement of the change in the diameter of the chest below the ribcage can provide additional accuracy to the measurement. Monitoring changes in the diameter of the chest below the ribcage can account for diaphragm delivered breathing where the contraction and relaxation of the diaphragm muscle causes the organs of the abdomen to be pushed down and outwards, thereby increasing the available volume of the lungs.

Monitoring and analyzing respiratory characteristics can be particularly useful in athletic applications, as there is a direct link between performance and an athlete's processing of oxygen and carbon dioxide. For example, in many athletic training situations, it is helpful to know when the athlete's body transitions between aerobic exercise and anaerobic exercise, sometimes referred to as the athlete's ventilatory threshold. Crossing over the ventilatory threshold level is an indicator of pending performance limitations during sport activities. For example, it can be beneficial for athletes to train in the anaerobic state for limited periods of time. However, for many sports, proper training requires only limited periods of anaerobic exercise interrupted by lower intensity aerobic exercises. It is difficult for an athlete to determine which state, anaerobic or aerobic, he or she is in without referencing physiological characteristics such as respiratory characteristics. Therefore, respiratory monitoring and data processing can provide substantial benefits in athletic training by allowing for accurate and substantially instantaneous measurements of the athlete's exercise state. Changes in an athlete's ventilatory threshold over time, as well as patterns of tidal volume during post-exercise recovery, can be valuable to measure improvements in the athlete's fitness level over the course of a training regime. Respiratory monitoring can further allow for monitoring and analyzing changes in a subject's resting metabolic rate.

A second ventilatory threshold exists at the point when the load on the body is such that the pulmonary ventilation is no longer sufficient to support life sustainably. Dwelling too long in this state will lead to collapse and so determination of this point can be of value in medical applications, and particularly to first responders and other emergency response personnel.

As indicated above, the present invention is directed to a method and system for creating an unprecedented physiologic database or library that can be indexed to at least health, disease, patient characteristics, and acute medical crises. The database can be structured and continuous/pseudo-continuous modeling statistics applied to provide novel insight into the natural history of disease and the response to treatment, and to ultimately improve care delivery. The database can also be an athletic performance database or library that can be indexed to performance or progress of an athlete or group of athletes, as well as standing relative to other athletes. The database can also be indexed to dangerous athletic conditions, such as, for example, exhaustion or overheating.

The database (and information derivation approach), coupled with the wealth of relevant/real-world data will enable otherwise impossible insight into epidemiology, natural history of various diseases, and the impact of treatment, will enable better insight into true cost of disease and treatment, and will ultimately improve patient medical care. The approach will also provide better insight into performance and progress of an athlete or group of athletes, as well as standing relative to other athletes.

According to the invention, various conventional monitoring systems can be employed within the scope of the invention to provide the physiological, performance, and anatomical information to create the physiologic databases or the athletic performance databases of the invention. The signals acquired by conventional systems, however, often only reflect one or a few physiological or performance characteristics (e.g., respiratory rate, blood pressure, temperature, speed, and/or heart rate). Thus, to create an in-depth library of physiologic or athletic performance data, multiple systems would need to be employed.

One preferred physiological monitoring system and associated method that is adapted to provide a plurality of signals representing multiple physiological and anatomical characteristics and parameters is disclosed in co-pending U.S. Patent Application No. 61/275,575, filed September 1, 2009, and co-pending U.S. Patent Application No. 12/869,582, filed concurrently herewith.

One preferred athletic performance monitoring system and associated method that is adapted to provide a plurality of signals representing multiple performance characteristics and parameters is disclosed in commonly owned U.S. Patent Application No. 11/892,023, titled "Sports Electronic Training System, and Applications Thereof," commonly owned U.S. Patent Application No. 12/467,944, titled "Portable Fitness Monitoring Systems, and Applications Thereof," and commonly owned U.S. Patent Application No. 12/836,421, titled "Fitness Monitoring Methods, Systems, and Program Products, and Applications Thereof,".

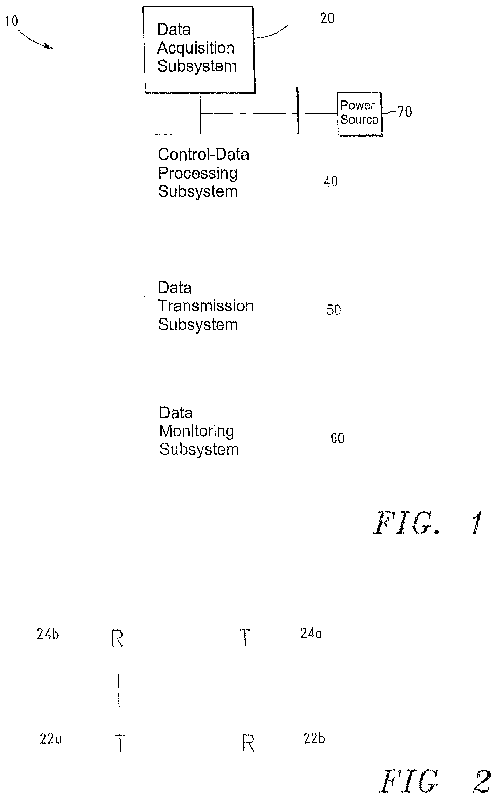

Referring first to Fig. I, there is shown a schematic illustration of one embodiment of a physiology monitoring system of the invention. As illustrated in Fig. 1, the physiology monitoring system 10 preferably includes a data acquisition subsystem 20, a control-data processing subsystem 40, a data transmission subsystem 50, a data monitoring subsystem 60, and a power source 70, such as a battery.

### Data Acquisition Subsystem

In accordance with one embodiment of the invention, the data acquisition subsystem 20 includes means for acquiring anatomical parameters that can be employed to determine at least one respiratory characteristic, more preferably a plurality of respiratory characteristics, in cooperation with control-data processing subsystem 40, and, in some embodiments, data monitoring subsystem 60. The anatomical parameters may include changes in (or displacements of) the anteroposterior diameters of the rib cage and abdomen, and axial displacement of the chest wall. The means for acquiring the noted parameters, e.g., sensors. The sensors can include paired electromagnetic coils or magnetometers.

Although the present invention is described herein in terms of magnetometers and magnetometer systems, it is understood that other types of sensor systems capable of measuring changes in distance between two or more sensors in the system can be used in place of, or in addition to, magnetometers. Specifically, the invention is not limited to the use of electromagnetic coils or magnetometers to measure changes in the anteroposterior diameters of the rib cage and abdomen, and axial displacement of the chest wall. Various additional means and devices that can be readily adapted to measure the noted anatomical parameters can be employed within the scope of the invention. Such means and devices include, without limitation, Hall effect sensors and electronic compass sensors. Wireless sensors with the capability of measuring time delay in a signal sent from one sensor to another and thereby determine the distance between the two sensors can be substituted for or provided in addition to magnetometers in accordance with the present invention.

According to the invention, at least two magnetometers can be employed to measure the noted subject parameters (or displacements). In some embodiments of the invention, two pairs of magnetometers are employed. In some embodiments, more than two pairs of magnetometers are employed.

Referring now to Fig. 2, there is shown one embodiment of a dual-paired electromagnetic coil arrangement for detecting and measuring displacement(s) of the rib cage, abdomen, and chest wall. As illustrated in Fig. 2, the electromagnetic coils include first transmission and receive coils 22a, 22b, and second transmission and receive coils 24a, 24b. In Fig. 2, the letter T designates the transmission coils and the letter R designates the receiving coils, however, the coils are not limited to such designations. The electromagnetic coils of embodiments of the present invention are described as "receiving" or "transmitting", however each receiving coil can alternatively and independently be a transmitting coil, and each transmitting coil can alternatively and independently be a transmitting coil..

Details of the noted arrangement and associated embodiments (discussed below) are set forth in co-pending U.S. Patent Application No. 12/231,692, filed September 5, 2008, co-pending U.S. Patent Application No. 61/275,576, filed September 1, 2009, and co-pending U.S. Patent Application No. 12/869,576, filed concurrently herewith, each of which, as indicated above.

As set forth in the noted applications, in some embodiments of the invention, at least receive coil 24b is adapted to receive coil transmissions from each of transmission coils 22a, 24a (i.e., at least receive coil 24b may be a dual function coil, where "dual function coil" refers to a coil capable of receiving transmissions from a plurality of different transmission coils). In some embodiments, each receive coil 22b, 24b is adapted to receive transmissions from each transmission coil 22a, 24a.

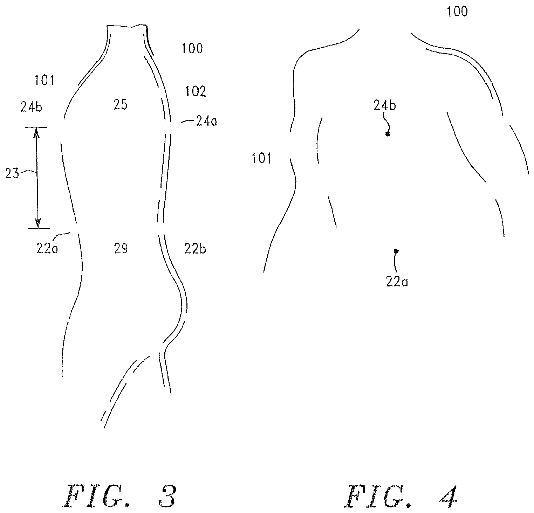

Referring now to Figs. 3-5, there is shown the position of coils 22a, 22b, 24a, 24b on a subject or patient 100, in accordance with one embodiment of the invention. As illustrated in Figs. 3-5, first transmission coil 22a is preferably positioned on front 101 of subject 100 proximate the umbilicus of subject 100, and first receive coil 22b is preferably positioned proximate the same axial position, but on back 102 of subject 100. Second receive coil 24b is preferably positioned on front 101 of subject 100 proximate the base of the sternum, and second transmission coil 24a is preferably positioned proximate the same axial position, but on back 102 of subject 100.

As set forth in co-pending U.S. Patent Application No. 12/231,692, the positions of transmission coils 22a, 24a and receive coils 22b, 24b can be reversed (i.e., transmission coil 22a and receive coil 24b can be placed on back 102 of subject 100 and transmission coil 24a and receive coil 22b can be placed on front 101 of subject 100. Both transmission coils 22a and 24a can also be placed on front 101 or back 102 of subject 100 and receive coils 22b and 24b can be placed on the opposite side.

Referring back to Fig. 3, an arrow 23 represents the chest wall or, in this instance, the xiphi-umbilical distance (Xi) that is monitored. An arrow 25 represents the monitored rib cage distance, while an arrow 29 represents the monitored abdominal distance.

In accordance with one embodiment of the invention, wherein coil 24b is a dual function coil, as subject or patient 100 breathes, displacement(s) of the rib cage and abdomen (i.e., changes in the distance between each pair of coils 22a, 22b and 24a, 24b, denoted, respectively, by arrow 29 and arrow 25), is determined from measured changes in voltage between paired coils 22a, 22b and 24a, 24b. The axial displacement of the chest wall, denoted by arrow 23, (e.g., xiphi-umbilical distance (Xi)), is also determined from measured changes in voltage between transmission coil 22a and receive coil 24b.

As indicated above, in some embodiments of the invention, more than two pairs of electromagnetic coils can be employed. As set forth in co-pending U.S. Patent Application No. 61/275,575, filed September 1, 2009, and co-pending U.S. Patent Application No. 12/869,582, filed concurrently herewith, adding additional pairs of electromagnetic coils in anatomically appropriate positions on a subject provides numerous significant advantages over dual-paired coil embodiments. Among the advantages is the provision of additional (and pertinent) data and/or information regarding chest wall movement(s) and the relationship(s) thereof to respiratory activity and respiratory associated events, such as speaking, sneezing, laughing, and coughing.

Further, the multiple single, cross, and interaction axes of the electromagnetic coil transmissions that result from the additional coils (and placement thereof) provide highly accurate quantification of changes in chest wall volume, and facilitate three-dimensional modeling of chest wall shape and movement of ambulatory subjects, and the evaluation and quantification of ventilatory mechanics, e.g., synchronous and asynchronous movement of the chest wall compartments.

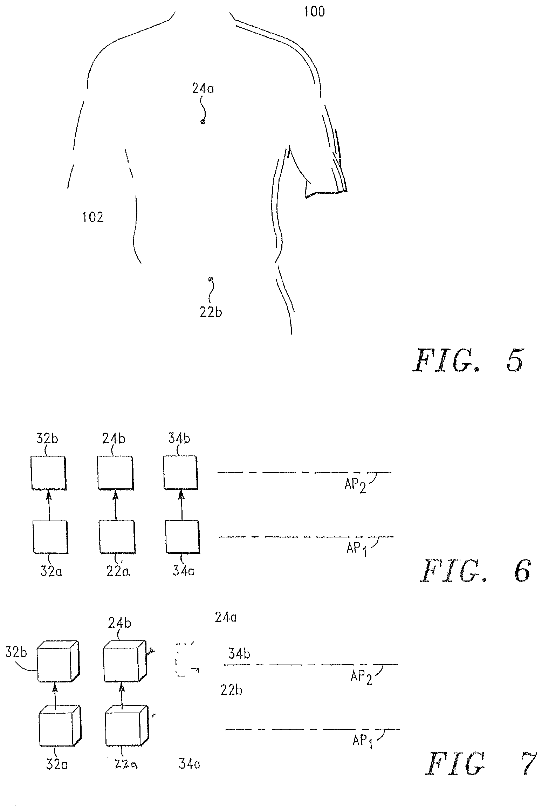

Referring now to Figs. 6-17, the multiple-paired coil embodiments of the invention will now be described in detail. It is, however, to be understood that the invention is not limited to the multiple-paired coil embodiments described herein. As will be appreciated by one having ordinary skill in the art, the multiple-paired coil embodiments can include any number of additional electromagnetic coils (e.g., 3, 4, 5, 6, 7, 8, 9, 10). For example, in embodiments using three magnetometers, for example, electromagnetic coils, it is understood that the three electromagnetic coils can function as multiple pairs. Specifically, referring to the coils as first, second, and third coils, the first coil can form a pair with the second coil and the first coil can also form a pair with the third coil. In addition, the second coil can also form a pair with the third coil. Thus, a magnetometer system utilizing three electromagnetic coils can be configured to form one, two, or three pairs. Each of the first, second, and third coils can be configured to transmit signals, receive signals, or to both receive and transmit signals. A magnetometer can communicate with a plurality of other magnetometers, and therefore a particular magnetometer can form a part of more than one pair. The position of the additional coils and the function thereof can also be readily modified and/or adapted for a particular application within the scope of the present invention.

Referring first to Figs. 6-8, there is shown one embodiment of the multiple-paired coil embodiment of the invention. As illustrated in Fig. 7, the noted embodiment similarly includes electromagnetic coils 22a, 22b, 24a, 24b. According to the invention, any of the aforementioned dual-paired coil embodiments associated with coils 22a, 22b, 24a, 24b can be employed with the multiple-paired coil embodiments of the invention.

As also illustrated in Figs. 6 and 7, the multiple-paired coil embodiment can further include at least two additional pairs of electromagnetic coils: third transmission coil 32a, third receive coil 32b, fourth transmission coil 34a, and fourth receive coil 34b.

In some embodiments of the invention, at least one of the two additional receive coils 32b, 34b is a dual function coil and, hence, adapted to receive transmissions from each of transmission coils 32a, 22a, 34a. In some embodiments, each receive coil 32b, 34b is adapted to receive transmissions from each transmission coil 32a, 22a, 34a.

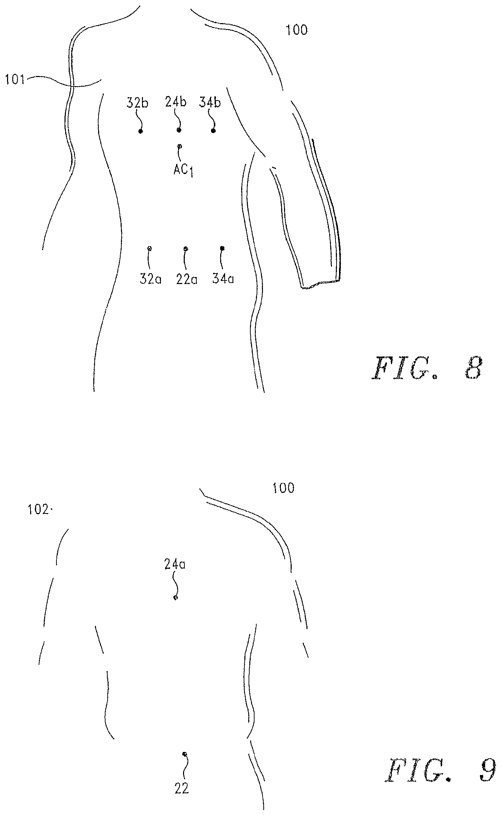

Referring now to Figs. 8 and 9, there is shown the position of coils 22a, 22b, 24a, 24b, 32a, 32b, 34a, 34b on a subject or patient 100, in accordance with one embodiment of the invention. As illustrated in Figs. 8 and 9, first transmission coil 22a is preferably positioned on front 101 of subject 100 proximate the umbilicus of subject 100, and first receive coil 22b is preferably positioned proximate the same axial position, but on back 102 of subject 100. Second receive coi124b is preferably positioned on front 101 of subject 100 proximate the base of the sternum, and second transmission coil 24a is positioned proximate the same axial position, but on back 102 of subject 100.

Third transmission coil 32a is preferably positioned on front 101 of subject 100 and axially spaced to the right of first transmission coil 22a. Fourth transmission coil 34a is preferably positioned on front 101 of subject 100 and axially spaced to the left of first transmission coil 22a. In the illustrated embodiment, each transmission coil 32a, 22a, 34a is preferably positioned proximate the same axial plane (denoted "AP₁" in Figs. 6 and 7).

Third receive coil 32b is preferably positioned on front 101 of subject 100 and axially spaced to the right of second receive coil 24b. Fourth receive coil 34b is preferably positioned on front 101 of subject 100 and axially spaced to the left of second receive coil 24b. Preferably, each receive coil 32b, 24b, 34b is similarly positioned proximate the same axial plane (denoted "AP₂" in Figs. 6 and 7).

As will readily be appreciated by one having ordinary skill in the art, the axial spacing of coils 32a, 32b, 34a, 34b will, in many instances, be dependant on the body size and structure of the subject, e.g., adult, female, male, adolescent. The distance between and amongst the coils can also vary with the degree of measurement precision required or desired.

As indicated above, a significant advantage of the multiple-paired coil embodiments of the invention is the provision of multiple single, cross, and interaction coil transmission axes that facilitate three-dimensional modeling of chest wall shape and movement of ambulatory subjects, and evaluation and quantification of ventilatory mechanics, e.g., synchronous and asynchronous movement of the chest wall compartments.

A further significant advantage of the multiple-paired coil embodiments of the invention is that real-time, three-dimensional models of the chest wall can be created by simultaneous monitoring of the chest wall with the multiple-paired coils of the invention.

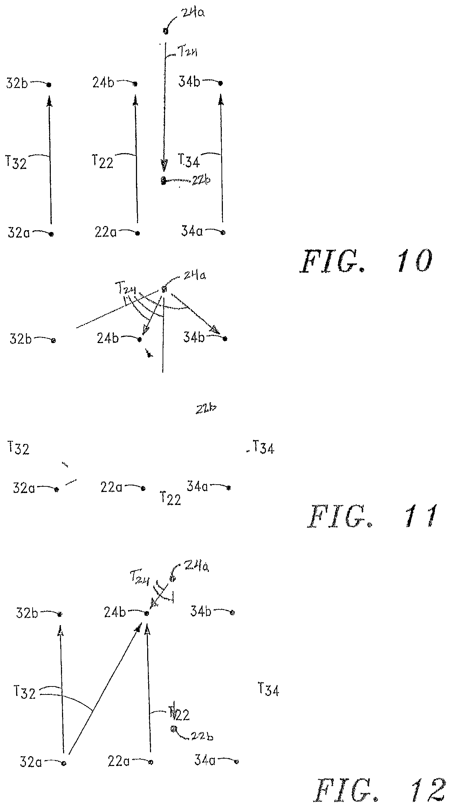

Referring now to Figs. 10-12, there are shown several schematic illustrations of coil transmission axes provided by three multiple-paired coil embodiments of the invention. Referring first to Fig. 10, there is shown one embodiment, wherein each receive coil 32b, 24b, 34b, 22b is a single function coil. Receive coil 32b is adapted to receive a transmission T₃₂ from transmission coil 32a. Receive coil 24b is adapted to receive a transmission T₂₂ from transmission coil 22a. Receive coil 34b is adapted to receive a transmission T₃₄ from transmission coil 34a. Receive coil 22b is adapted to receive a transmission T₂₄ from transmission coil 24a.

Referring now to Fig, 12, there is shown another embodiment, wherein receive coil 24b is a dual function coil. Receive coil 32b is adapted to receive transmission T₃₂ from transmission coil 32a, receive coil 34b is adapted to receive transmission T₃₄ from transmission coil 34a, and receive coil 22b is adapted to receive transmission T₂₄ from transmission coil 24a. Receive coil 24b is, however, adapted to receive transmission T₃₂ from transmission coil 32a, transmission T₂₂ from transmission coil 22a, transmission T₃₄ from transmission coil 34a, and transmission T₂₄ from transmission coil 24a.

In a further embodiment, illustrated in Fig. 11, each receive coil 32b, 24b, 34b, 22b is a dual function coil. As illustrated in Fig. 11, receive coil 32b is adapted to receive transmission T₃₂ from transmission coil 32a, transmission T₂₂ from transmission coil 22a, transmission T₃₄ from transmission coil 34a, and transmission T₂₄ from transmission coil 24a. Receive coils 24b, 34b, and 22b are also adapted to receive transmission T₃₂ from transmission coil 32a, transmission T₂₂ from transmission coil 22a, transmission T₃₄ from transmission coil 34a, and transmission T₂₄ from transmission coil 24a.

The noted multiple-paired coil embodiments significantly enhance the available data and information associated with chest wall movement and, hence, respiratory activity and respiratory associated events. The additional data and information also facilitates the evaluation and quantification of ventilatory mechanics, e.g., synchronous and asynchronous movement of the chest wall compartments.

The supplemental coil transmissions (or signals) can also be readily employed to reduce or eliminate the frequency and impact of magnetic field interference and artifacts, which are commonly encountered in electromagnetic coil systems.

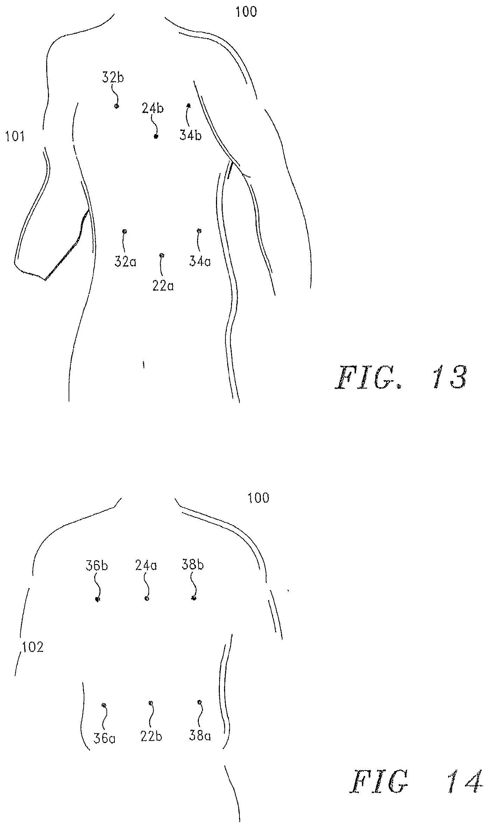

As indicated above, the multiple-paired coil embodiments of the invention are not limited to the embodiment described above, wherein two additional pairs of electromagnetic coils are uniformly positioned on the front of a subject. Referring now to Figs. 13-17, there are shown additional multiple-paired coil embodiments of the invention.

Referring first to Fig. 13, there is shown a multiple-paired coil embodiment, wherein the two additional coil pairs 32a, 32b, and 34a, 34b are non-uniformly positioned on front 101 of subject 100. As indicated, the additional coil pairs can be positioned at any appropriate (or desired) positions on the torso of subject 100.

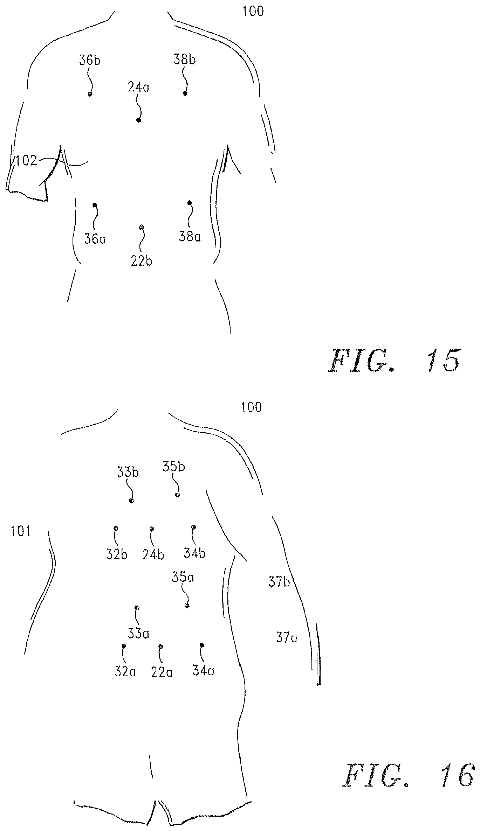

Additional paired coils (e.g., transmission coil 36a paired with receive coil 36b, and transmission coil 38a paired with receive coil 38b) can also be positioned on back 102 of subject 100, as illustrated in Fig. 14. Coils 36a, 36b, 38a, 38b can be positioned uniformly, as shown in Fig. 14, or non-uniformly, as illustrated in Fig. 15.

Referring now to Figs. 16-17, there is shown another multiple-paired coil embodiment, wherein additional paired coils are positioned on the torso of subject 100. As illustrated in Fig. 16, additional paired coils (e.g., transmission coil 33a paired with receive coil 33b, and transmission coil 35a paired with receive coil 35b) can be positioned on front 101 of subject 100. In the noted embodiment, transmission coil 33a is preferably positioned above and between transmission coils 32a and 22a, and transmission coil 35a is preferably positioned above and between transmission coils 22a and 34a. Receive coil 33b is preferably positioned above and between receive coils 32b and 24b, and receive coil 35b is preferably positioned above and between receive coils 24b and 34b.

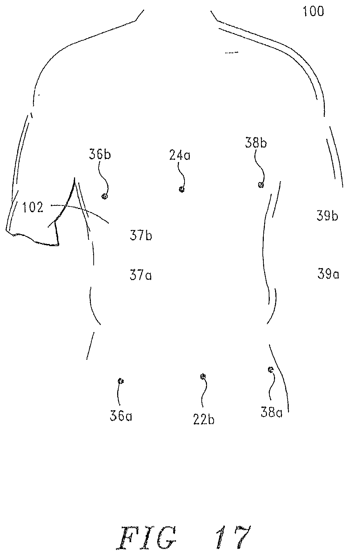

As illustrated in Figs. 16 and 17, additional paired coils (e.g., transmission coil 37a paired with receive coil 37b, and transmission coil 39a paired with receive coil 39b) can be also positioned on opposite sides of the subject 100.

Additionally, the transmission coils and receive coils disclosed herein need not necessarily be paired one-to-one. For example, a single receive coil may be configured to receive transmissions from multiple transmission coils, and a single transmission coil may be configured to transmit to multiple receive coils.

As indicated above, the multiple-paired coil embodiments of the invention are not limited to the multiple-paired coil embodiments shown in Figs. 6-17. It is again emphasized that the multiple-paired coil embodiments can include any number of additional pairs of electromagnetic coils. Further, the position of the additional coils and the function thereof can also be readily modified and/or adapted for a particular application within the scope of the present invention.

In some embodiments of the invention, the data acquisition subsystem 20 can include means for directly monitoring the orientation and/or movement of subject 100, e.g., spatial parameters. According to the invention, various conventional means can be employed to monitor or measure subject orientation and movement, including optical encoders, proximity and Hall effect switches, laser interferometry, accelerometers, gyroscopes, global positioning systems (GPS), and/or other spatial sensors.

In one embodiment, the means for directly monitoring the orientation and movement of a subject includes at least one multi-function inertial sensor, e.g., 3-axis accelerometer and 3-axis gyroscope. As is well known in the art, orientation and motion of a subject can be readily determined from the signals or data transmitted by a multi-function accelerometer.

According to the invention, the accelerometer can be disposed in any anatomically appropriate position on a subject. In one embodiment of the invention, an accelerometer (denoted "AC₁" in Fig. 8) is disposed proximate the base of the subject's sternum. Accelerometers and other inertial sensors can assist in determining the rate of speed at which a subject is traveling, the amount of energy an athlete is exerting during physical exercise, whether the athlete is airborne, and various information related to gait characteristics of the subject.

In some embodiments of the invention, data acquisition subsystem 20 includes at least one additional physiological sensor (preferably, a plurality of additional physiological sensors) adapted to monitor and record one or more physiological characteristics associated with monitored subject 100. The physiological sensors can include, without limitation, sensors that are adapted to monitor and record electrical activity of the brain, heart, and other muscles (e.g., EEG, ECG, EMG), pulse rate, blood oxygen saturation level (e.g., SpO₂), skin temperature, and core temperature. Physiological parameters measured and/or calculated may include, for example, heart rate, respiration rate, blood oxygen level, blood flow, hydration status, calories burned, muscle fatigue, and/or body temperature.

Exemplary physiological sensors are disclosed in U.S. Patent No. 6,551,252, U.S. Patent No. 7,267,652, and co-pending U.S. Patent Application No. 11/764,527, filed June 18, 2007.

According to exemplary embodiments of the invention, the additional sensors can be disposed in a variety of anatomically appropriate positions on a subject. By way of example, a first sensor (e.g., a pulse rate sensor) can be disposed proximate the heart of subject 100 to monitor pulse rate, and a second sensor (e.g., a microphone) can be disposed proximate the throat of subject 100 to monitor sounds emanating therefrom (e.g., sounds reflecting coughing).

As indicated above, data acquisition subsystem 20 can also include one or more audio sensors, such as, for example, a microphone, for monitoring sounds generated by a monitored subject, and a speaker to enable two-way communication by and between the monitored subject and a monitoring station or individual.

According to embodiments of the invention, the paired coils (e.g., electromagnetic coils 22a, 22b, 24a, 24b, and the aforementioned additional sensors) can be positioned on or proximate a subject by various suitable means. Thus, in some embodiments, the paired coils and/or additional sensors can be directly attached to the subject.

In some embodiments, the paired coils, additional sensors, processing and monitoring systems (e.g., LDUs, if employed) are embedded in or carried by a wearable garment or item that can be comfortably worn by a monitored subject. The associated wiring, cabling, and other power and signal transmission apparatuses and/or systems can also be embedded in the wearable garment.

According to embodiments of the invention, the wearable monitoring garment can be one or more of a variety of garments, such as a shirt, vest or jacket, belt, cap, patch, and the like. A suitable wearable monitoring garment (a vest) is illustrated and described in co-pending U.S. Patent Application No. 61/275,576, filed September 1, 2009, co-pending U.S. Patent Application No. 12/869,576, filed concurrently herewith, co-pending U.S. Patent Application No. 61/275,633, filed September 1, 2009, and co-pending U.S. Patent Application No. 12/869,627, filed concurrently herewith.

### Control-Data Processing Subsystem

According to the present invention, control-data processing subsystem 40 can include programs, instructions, and associated algorithms for performing the methods of the invention, including control algorithms and associated parameters to control data acquisition subsystem 20 and, hence, the paired electromagnetic coils, e.g., coils 22a, 22b, 24a, 24b, 32a, 32b, 34a, 34b and the function thereof, and the transmission and receipt of coil transmissions, e.g., transmissions T₃₂, T₂₂, T₃₄, and T₂₄, as well as data transmission subsystem 50 and data monitoring subsystem 60. Such is discussed in detail below.

Control-data processing subsystem 40 is further programmed and adapted to retrieve and process coil transmissions or signals from the electromagnetic coils (e.g., coils 22a, 22b, 24a, 24b, 32a, 32b, 34a, 34b) in order to determine physiological information associated with monitored subject 100, to retrieve, process, and interpret additional signals transmitted by additional spatial parameter and physiological sensors (discussed below), and to transmit selective coil data, physiological and spatial parameters, physiological characteristics, and subject information to data monitoring subsystem 60.

In a preferred embodiment of the invention, control-data processing subsystem 40 further includes at least one "n-degrees-of-freedom" model or algorithm for determining at least one respiratory characteristic (e.g., V_{T}) from the retrieved coil transmissions or signals (e.g., measured displacements of the rib cage, abdomen, and chest wall).

Control-data processing subsystem 40 also preferably includes suitable algorithms that are designed and adapted to conduct multivariable analyses of data acquired by data acquisition subsystem 20, (e.g., coil transmissions and signals transmitted by additional spatial parameter and physiological sensors, as discussed below).

In one embodiment, control-data processing subsystem 40 includes one or more "three-degrees-of freedom" models or algorithms for determining at least one respiratory characteristic (preferably, a plurality of respiratory characteristics) from the retrieved coil transmissions (or signals). Preferred "three-degrees-of freedom" models (or algorithms) are set forth in co-pending U.S. Patent Application No. 12/231,692.

In some embodiments, control-data processing subsystem 40 is further programmed and adapted to assess physiological characteristics and parameters by comparison with stored physiological benchmarks. Control-data processing subsystem 40 can also be programmed and adapted to assess respiratory and spatial characteristics and parameters by comparison with stored respiratory and spatial benchmarks Control-data processing subsystem 40 can generate status signals if corresponding characteristics or parameters are present. The benchmarks may indicate, for example, adverse conditions or fitness goals, and the status signals may include warnings or alarms.

Control-data processing subsystem 40 also preferably includes suitable algorithms that are designed and adapted to determine respiratory characteristics, parameters, and statuses from measured multiple, interactive chest wall displacements. The algorithms are also preferably adapted to discount measured chest wall displacements that are associated with non-respiration movement, e.g., twisting of the torso, to enhance the accuracy of respiratory characteristic (and/or parameter) determinations.

Control-data processing subsystem 40 additionally preferably includes suitable programs, algorithms, and instructions to generate three-dimensional models of subject's chest wall from the measured multiple, interactive chest wall displacements.

According to the invention, various programs and methods known in the mathematical arts (e.g., differential geometric methods) can be employed to process the signals (reflecting the chest wall distances and displacement) into a representation of the shape of the torso. Indeed, it is known that providing sufficient distances defined on a two dimensional surface (a metric) permit the shape of the surface to be constructed in a three dimensional space. See, e.g., Badler, et al., "Simulating Humans: Computer Graphics, Animation, and Control", (New York: Oxford University Press, 1993) and DeCarlo, et al., "Integrating Anatomy and Physiology for Behavior Modeling", Medicine Meets Virtual Reality 3 (San Diego, 1995).

Preferably, in some embodiments of the invention, control-data processing subsystem 40 is further programmed and adapted to determine additional and, in some instances, interrelated anatomical parameters, such as bending, twisting, coughing, etc., from the measured multiple, interactive chest wall displacements. In one embodiment, control-data processing subsystem 40 is programmed and adapted to compare retrieved coil transmissions reflecting measured chest wall displacements with stored selective combinations of coil transmissions and chest wall parameters that are associated therewith (e.g., "normal respiration and bending", "normal respiration and coughing").

By way of example, in one embodiment, a first chest wall parameter (CWP₁) defined as (or reflecting) "normal respiration and twisting of the torso" is stored in control-data processing subsystem 40. The coil transmissions and data associated with the first chest wall parameter (CWP₁) include transmissions T₃₂, T₂₂, T₃₄, and T₂₄ received by receive coil 24b that can represent displacements x, y, and z.

During monitoring of subject 100, similar coil transmissions may be received by receive coil 24b. Control-data processing subsystem 40 then compares the detected (or retrieved) transmissions to the stored transmissions and chest wall parameters associated therewith to determine (in real-time) the chest wall movement and, hence, respiratory activity based thereon; in this instance "normal respiration and twisting of the torso".

In some embodiments, the signals transmitted by the accelerometer (e.g., spatial parameter signals) are employed with the detected coil transmissions to determine and classify chest wall movement and associated respiratory activity of the monitored subject. In the noted embodiments, each stored chest wall parameter also includes spatial parameter signals associated with the chest wall parameter (e.g., normal respiration and twisting of the torso). According to the invention, control-data processing subsystem 40 is adapted to compare retrieved coil transmissions and spatial parameter signals to the stored transmissions and spatial parameter signals, and the chest wall parameters associated therewith, to determine the chest wall movement and, hence, respiratory activity based thereon.

In some embodiments, the spatial parameter signals are used to generate a spatial model of the subject. The spatial model can be two-dimensional or three-dimensional, and can reflect the real-time orientation and movement of the subject. The spatial model can be displayed to provide the subject or another with a representation of the real-time orientation and movement of the subject.

In some embodiments of the invention, control-data processing subsystem 40 is programmed and adapted to determine chest wall movement and respiratory activity based on retrieved coil transmissions, spatial parameter signals, and audio signals. In the noted embodiments, data acquisition subsystem 20 can also include an audio sensor, such as, e.g., a microphone, that is disposed in an anatomically appropriate position on a subject, e.g., proximate the throat.

According to the invention, each stored chest wall parameter also includes at least one audio parameter (e.g., > N db, based on the audio signal) that is associated with the chest wall parameter (e.g., normal respiration and coughing). Suitable speech and cough parameters (and threshold determinations) are set forth in U.S. Patent No. 7,267,652, issued September 11, 2007.

Upon receipt of coil transmissions, spatial parameter signals, and audio signals, control-data processing subsystem 40 compares the retrieved coil transmissions, spatial parameter signals, and audio signals to the stored transmissions, spatial parameter signals, and audio parameters, and the chest wall parameters associated therewith, to determine the chest wall movement and respiratory activity based thereon (e.g., normal respiration and coughing).

In some embodiments of the invention, control-data processing subsystem 40 is programmed and adapted to determine fitness activity based on retrieved coil transmissions, spatial parameter signals, and audio signals. In the noted embodiments, data acquisition subsystem 20 may also include an audio sensor, such as, for example, a microphone, that is disposed in an anatomically appropriate position on a subject (e.g., proximate the throat).

Upon receipt of coil transmissions, spatial parameter signals, and audio signals, control-data processing subsystem 40 compares the retrieved coil transmissions, spatial parameter signals, and audio signals to the stored transmissions, spatial parameter signals, and audio parameters, and the chest wall parameters associated therewith, to determine a fitness activity of the subject (e.g., running, jogging, stretching, swimming, performing push-ups, performing sit-ups, performing chin-ups, performing arm curls, playing basketball, playing baseball, or playing soccer).

### Data Monitoring Subsystem

According to embodiments of the invention, data monitoring subsystem 60 is designed and adapted to receive and, in some embodiments, to selectively monitor coil transmissions or signals (e.g., transmissions T₃₂, T₂₂, T₃₄, and T₂₄) and to display parameters associated therewith (e.g., displacement(s) along a selective axis), and/or a chest wall parameter (e.g., CWP₁), and/or a respiratory characteristic (e.g., V_{T}) or event.

Data monitoring subsystem 60 is further preferably designed and adapted to display selective subject parameters, characteristics, information, and warnings or alarms. Data monitoring subsystem 60 can also be adapted to display data or broadcast data aurally. The aurally presented data can be voice messages, music, or other noises signifying an event. Data monitoring subsystem 60 can be adapted to allow headphones or speakers to connect to the data monitoring subsystem, either wireless or wired, to broadcast the aural data. Data monitoring subsystem 60 can be adapted to include a display, or to allow a display to connect to the data monitoring subsystem, to display the data. Such display can include, for example, a liquid crystal display (LCD), a plasma display, a cathode ray tube (CRT) display, a light emitting diode (LED) display, or an organic light emitting diode (OLED) display.

In some embodiments of the invention, data monitoring subsystem 60 is also adapted to receive and, in some embodiments, selectively monitor spatial parameter signals and signals transmitted by additional anatomical and physiological sensors (e.g., signals indicating skin temperature, or SpO₂) and to display parameters and information associated therewith. The parameters can be associated with an athlete's physical activity. Physical or anatomical parameters measured and/or calculated may include, for example, time, location, distance, speed, pace, stride count, stride length, stride rate, and/or elevation. Physiological parameters measured and/or calculated may include, for example, heart rate, respiration rate, blood oxygen level, blood flow, hydration status, calories burned, muscle fatigue, and/or body temperature. In an embodiment, performance parameters may also include mental or emotional parameters such as, for example, stress level or motivation level. Mental and emotional parameters may be measured and/or calculated directly or indirectly either through posing questions to the athlete or by measuring things such as, for example, trunk angle or foot strike characteristics while running.

In some embodiments of the invention, data monitoring subsystem 60 includes a local electronic module or local data unit (LDU). The term "local" as used in connection with an LDU is intended to mean that the LDU is disposed close to the electromagnetic coils, such as on or in a wearable garment containing the coils (discussed in detail below).

In some embodiments of the invention, the LDU is preferably adapted to receive and monitor coil transmissions (or signals), to preprocess the coil transmissions, to store the coil transmissions and related data, and to display selective data, parameters, physiological characteristics, and subject information.

In some embodiments, the LDU is also adapted to receive and monitor the spatial parameter transmissions (or signals) and additional signals transmitted by additional anatomical and physiological sensors (if employed), to preprocess the signals, to store the signals and related data, and to display selective data, physiological and spatial parameters, physiological characteristics, and subject information via a variety of media, such as a personal digital assistant (PDA), a mobile phone, and/or a computer monitor, etc.

In some embodiments, the LDU includes a remote monitor or monitoring facility. In these embodiments, the LDU is further adapted to transmit selective coil and sensor data, physiological parameters and characteristics, spatial parameters, and subject information to the remote monitor or facility.

In some embodiments of the invention, the LDU includes the features and functions of control-data processing subsystem 40 (e.g., an integral control-processing/monitoring subsystem) and, hence, is also adapted to control data acquisition subsystem 20. The LDU is thus adapted to control the paired coils that are employed, to determine selective physiological characteristics and parameters, to assess physiological characteristics and parameters for adverse conditions, and to generate warnings or alarms if adverse characteristics or parameters are present.

Suitable LDUs are described in co-pending International Application No. PCT/US2005/021433 (Pub. No. WO 2006/009830 A2), published January 26, 2006.

In some embodiments of the invention, monitoring subsystem 60 includes a separate, remote monitor or monitoring facility. According to embodiments of the invention, the remote monitor or facility is adapted to receive sensor data and information, physiological and spatial parameters, physiological characteristics, and subject information from control-data processing subsystem 40, and to display the selective coil sensor data and information, physiological and spatial parameters, physiological characteristics, and subject information via a variety of mediums, such as a PDA, computer monitor, etc.

### Data Transmission Subsystem

According to embodiments of the invention, various communication links and protocols can be employed to transmit control signals to data acquisition subsystem 20 and, hence, paired coils, and to transmit coil transmissions (or signals) from the paired coils to control-data processing subsystem 40. Various communication links and protocols can be employed to transmit data and information, including coil transmissions (or signals) and related parameters, physiological characteristics, spatial parameters, and subject information from control-data processing subsystem 40 to data monitoring subsystem 60.

In some embodiments of the invention, the communication link between data acquisition subsystem 20 and control-data processing subsystem 40 includes conductive wires or similar direct communication means. In some embodiments, the communication link between data acquisition subsystem 20 and control-data processing subsystem 40, as well as between control-data processing subsystem 40 and data monitoring subsystem 60, is a wireless link.

As set forth in co-pending U.S. Patent Application No. 61/275,587, filed September 1, 2009, and co-pending U.S. Patent Application No. 12/869,592, filed concurrently herewith, data transmission subsystem 50 of the invention can also include multimodal communications means, which enable effective communication to data monitoring subsystem 60 or other desired device or system to be acquired in a wide range of settings where a single radio mode would be ineffective.

In one embodiment, the multimodal communications means includes multiple radio subsystems, including, for example, Global System for Mobile Communications (GSM) and BlueTooth® systems.

Data transmission subsystem 50 may further include algorithms and programming to control the noted multimodal communications means. Such controls may include preprogrammed transmission of signals (e.g., transmission when the physiological characteristic represented by a signal is deemed relevant).

The multimodal communications means in conjunction with the real-time signal acquisition and processing capabilities discussed above creates a "smart" communications structure that maximizes battery efficiency, ensures connectivity across a range of environments, and enables data to be transmitted only when relevant.

As set forth in co-pending U.S. Patent Application No. 61/275,634, filed September 1, 2009, and co-pending U.S. Patent Application No. 12/869,625, filed concurrently herewith, the physiological monitoring systems and methods described above can readily provide a plurality of signals representing a multitude of key physiological, performance, and contextual parameters continuously (or pseudo-continuously), synchronously, and simultaneously.

Acquiring the signals continuously (or pseudo-continuously), synchronously, and simultaneously facilitates the creation of in-depth physiologic databases and libraries and provides context that enables the interpretation and analysis of the data to be more effective.

In one embodiment of the invention, the databases are continuously updated in real-time or pseudo real-time with detailed physiological or performance data from a large number of subjects in the general population. In one embodiment, individual subject data-packages are annotated with subject demographic and medical or athletic performance history data enabling subsets of the larger database to be evaluated. This database structure, coupled with continuous real-world data updates and regular and frequent data reduction and algorithm treatment, will provide an unprecedented physiologic/medical library for disease and treatment profiling, or athletic performance library for performance monitoring and comparison.

The total database can be evaluated for normative data values for, for example, cardiac activity, respiratory activity, and other physiologic parameters. The total database can also or alternatively be evaluated for normative data values for, for example, athletic performance characteristics or progression.

In one embodiment, subsets of the database are developed based on disease state, and pattern recognition algorithms are applied to determine signals representing disease worsening, improvement, response to treatment, etc. This function of the database provides substantial potential benefit for future pharmaceutical and biotechnology development by providing nonnative data for disease groups as well as novel potential treatment targets and endpoints for clinical trial work.

Additionally, specific subsets of patients within the larger disease definitions will likely become evident and this will result in more precise diagnoses and delivery of medical care and treatment.

In one embodiment, subsets of the database are developed based on attributes such as, for example, athletic performance level and type of sport played, and pattern recognition algorithms are applied to determine signals representing performance worsening, improvement, response to training, etc. This function of the database provides substantial potential benefit for future athletic performance training development by providing non-native data for athlete groups as well as novel potential fitness targets and goals for athletic performance optimization. The database can help an athlete track changes in various physical and performance characteristics over time, which can help the athlete determine which training methods or programs are most effective.

Additionally, information included in the database can be used to predict disease progression accurately by correlating a subject's present condition to historical data of disease progression in other subjects exhibiting similarities to the subject's present condition. Such predictions can be further personalized to a particular subject by comparison with demographic data of the other subjects.

Additionally, information included in the database can be used to predict athletic performance progression accurately by correlating an athlete's present performance level or characteristics to historical data of athletic performance progression in other athletes exhibiting similarities to the athlete's present performance level or characteristics. Such predictions can be further personalized to a particular subject by comparison with demographic data of the other athletes.

In some exemplary embodiments the database may be embodied in a single physical storage medium or unit, such as, for example, a hard drive, random access memory (RAM), read-only or rewritable optical discs such as, for example, compact disc read-only memory (CD-ROM), compact disc rewritable (CD-RW), and digital video disc (DVD), floppy discs, floptical discs, solid-state memory, and various other storage mediums and units that would be apparent to one of skill in the art.

In some exemplary embodiments, the database is embodied in multiple separate storage mediums, which may or may not be of the same type. The separate storage mediums can all be a part of one cohesive system, connected to one another and to an access unit, such as a computer. Such connection can be by wires or wirelessly. In some embodiments the separate storage units can be connected to various distinct systems, which may or may not be located remotely from one another. In such a case, the separate storage units can be connected to one another and at least one access unit via a network, such as an intranet or the Internet.

In some exemplary embodiments, an access unit used to access the database is directly connected to the database unit(s) (e.g., via wires or wirelessly). In some exemplary embodiments an access unit to access the database is located remotely from the database unit(s) and connects to the database via, for example, a network, such as an intranet or the Internet.

Additional advantages and applications of the present invention are apparent with reference to the systems and methods disclosed in U.S. Patent Application No. 12/869,625, filed concurrently herewith, U.S. Patent Application No. 12/869,582, filed concurrently herewith, U.S. Patent Application No. 12/869,576, filed concurrently herewith, U.S. Patent Application No. 12/869,585, filed concurrently herewith, U.S. Patent Application No. 12/869,592, filed concurrently herewith, U.S. Patent Application No. 12/869,627, filed concurrently herewith, and U.S. Patent Application No. 12/869,578, filed concurrently herewith.

Without departing from the scope of this disclosure, one of ordinary skill can make various changes and modifications to the invention to adapt it to various usages and conditions. As such, these changes and modifications are properly, equitably, and intended to be, within the full range of equivalence of the invention.

Further preferred embodiments of the invention are mentioned as follows:
1.A system for monitoring and recording parameters of a subject engaged in a physical activity, the monitoring system comprising:
   a monitoring garment adapted to cover at least a portion of a subject's torso;
   a sensor subsystem including a first sensor and a second sensor, wherein the first and second sensors are responsive to changes in distance therebetween, wherein the sensor subsystem is configured to generate and transmit a distance signal representative of the distance between the first and second sensors, wherein the first and second sensors are incorporated into the garment, and wherein the first and second sensors are proximate to a subject's chest region when the garment is worn by the subject; and
   a database configured to receive and store the distance signal, wherein the database is further configured to receive and store a second signal, and wherein the database is configured to compare information represented by the first signal with information represented by the second signal.
2.The system of embodiment 1, wherein the second signal is representative of at least one of a physiological parameter and a performance parameter.
3.The system of embodiment 1, wherein the database is further configured to receive and store the signals continuously, synchronously, and simultaneously.
4.The system of embodiment 1, wherein the first signal is stored in association with demographic information of the subject and performance history of the subject.
5.The system of embodiment 1, wherein the database comprises a database subset configured to determine if the information represented by the first signal shares a common attribute with the information represented by the second signal, and wherein, if it is determined an attribute is shared, the database subset is configured to associate the first signal and the second signal.
6.The system of embodiment 5, wherein the attribute is at least one of athletic performance level and type of sport.
7.The system of embodiment 1, wherein the first and second sensors comprise electromagnetic coils.
8.A database system for population modeling, the database system comprising:
   a data acquisition subsystem configured to generate and transmit a first signal; and
   a processor subsystem in communication with the data acquisition subsystem, wherein the processor subsystem is configured to receive the first signal and process the first signal to obtain a first parameter signal that is representative of a parameter of the subject and to transmit the first parameter signal to a database, the database being configured to receive and store the first parameter signal,
   wherein the database is configured to receive and store a second parameter signal representative of a parameter of a second subject, and wherein the database is configured to compare the first parameter signal with the second parameter signal to determine similarities between the parameter information contained in the first parameter signal and the second parameter signal.
9.The database system of embodiment 8, wherein the parameters comprise at least one of a physiological parameter and a performance parameter.
10.The database system of embodiment 8, wherein the data acquisition subsystem is further configured to generate and transmit a plurality of signals continuously, synchronously, and simultaneously, and wherein the processor subsystem is further configured to receive and transmit the plurality of signals continuously, synchronously, and simultaneously.
11.The database system of embodiment 8, wherein the database is further configured to store the first parameter signal in association with demographic information of the first subject and historical athletic performance data of the related subject.
12.The database system of embodiment 11 wherein the database comprises a database subset configured to determine if the first parameter signal and second parameter signal share a common attribute, and wherein, if it is determined that the first and second parameter signals share an attribute, the database subset is configured to associate the first parameter signal and the second parameter signal.
13.The database system of embodiment 12, wherein the attribute is at least one of athletic performance level and type of sport.
14.The database system of embodiment 8, wherein pattern recognition algorithms are applied to determine if a parameter signal represents at least one of performance worsening, performance improvement, and response to training.
15.The database system of embodiment 12, wherein the attribute is indicative of a dangerous condition.
16.The database system of embodiment 8, wherein the data acquisition subsystem further comprises a sensor positioned proximate to the subject's chest region.
17.The database system of embodiment 16, wherein the sensor comprises a pair of electromagnetic coils responsive to changes in a distance therebetween.
18. The database system of embodiment 17, wherein the signals represent a respiratory parameter indicated by a change in the distance between the pair of electromagnetic coils.
19.A method for modeling a population, the method comprising:
   receiving a first signal from a first subject;
   processing the first signal with a processor subsystem to obtain a first parameter signal that is representative of a parameter of the first subject;
   transmitting the first parameter signal to a database;
   associating the first parameter signal with at least one of demographic information and performance history of the first subject; and
   storing the first parameter signal in a database in association with at least one of demographic information of the first subject and performance history of the first subject,
   comparing the first signal with a second parameter signal in the database.
20.The method of embodiment 19, wherein the parameter is at least one of a physiological parameter and a performance parameter.
21.The method of embodiment 19, wherein the second parameter signal represents a second parameter of the first subject.
22.The method of embodiment 19, wherein the second parameter signal represents a parameter of a second subject.
23. The method of embodiment 22, further comprising:
   determining a common attribute among the first and second subjects based on the first and second parameter signals; and
   storing a database subset, wherein the database subset comprises the first and second parameter signals.
24.The method of embodiment 19, wherein receiving the first and second parameter signals comprises receiving the first and second parameter signals continuously, synchronously, and simultaneously.
25.The method of embodiment 23, wherein the common attribute is at least one of athletic performance level and type of sport.
26.The method of embodiment 23, wherein the common attribute is indicative of a dangerous condition.
27.The method of embodiment 19, further comprising applying pattern recognition algorithms to determine if the first parameter signal indicates a change in performance compared with a previously stored parameter signal from the first subject.

## Claims

1. A database system for processing athletic performance data, the database system comprising:
a data acquisition subsystem configured to generate and transmit a first signal; and
a processor subsystem in communication with the data acquisition subsystem, wherein the processor subsystem is configured to receive the first signal and process the first signal to obtain a first parameter signal that is representative of a physiological parameter or a performance parameter of a subject (100) and to transmit the first parameter signal to a database, the database being configured to receive and store the first parameter signal,
wherein the database is configured to receive and store a second parameter signal representative of a physiological parameter or a performance parameter of a second subject;
wherein the database system further comprises a control-data processing subsystem configured to compare the first parameter signal with the second parameter signal to determine similarities between the parameter information contained in the first parameter signal and the second parameter signal, and
wherein the database is configured to store the first parameter signal in association with demographic information of the subject (100) and historical athletic performance data of the subject (100);
and **characterized in that**
the control-data processing subsystem is configured to determine if the first parameter signal and second parameter signal share a common attribute, wherein the attribute is an athletic performance level or a type of sport,
and wherein, if it is determined that the first and second parameter signals share the attribute, the control-data processing subsystem is configured to store a database subset that comprises the first and second parameter signals.

2. The database system of claim 1, wherein pattern recognition algorithms are applied to determine if a parameter signal represents at least one of performance worsening, performance improvement, and response to training.

3. The database system of claim 1, wherein the data acquisition subsystem further comprises a sensor (24) positioned proximate to the subject's chest region.

4. The database system of claim 3, wherein the sensor (24) comprises a pair of electromagnetic coils (24a, 24b) responsive to changes in a distance therebetween.

5. The database system of claim 4, wherein the signals represent a respiratory parameter indicated by a change in the distance between the pair of electromagnetic coils (24a, 24b).

6. A method for processing athletic performance data, the method comprising:
receiving a first signal from a first subject (100);
processing the first signal with a processor subsystem to obtain a first parameter signal that is representative of a physiological parameter or a performance parameter of the first subject (100);
transmitting the first parameter signal to a database;
associating the first parameter signal with demographic information and performance history of the first subject (100);
storing the first parameter signal in the database in association with the demographic information of the first subject (100) and the performance history of the first subject (100);
comparing the first signal with a second parameter signal in the database, wherein the second parameter signal represents a physiological parameter or a performance parameter of a second subject;
and **characterized by**
determining a common attribute among the first and second subjects based on the first and second parameter signals, wherein the attribute is an athletic performance level or a type of sport; and
storing a database subset if it is determined that an attribute is shared, wherein the database subset comprises the first and second parameter signals.

7. The method of claim 6, further comprising applying pattern recognition algorithms to determine if the first parameter signal indicates a change in performance compared with a previously stored parameter signal from the first subject.

## Patentansprüche

1. Datenbanksystem zum Verarbeiten athletischer Performancedaten, das Datenbanksystem beinhaltend:
ein Datenerfassungssubsystem dazu ausgebildet, ein erstes Signal zu generieren und zu übermitteln; und
ein Prozessorsubsystem in Kommunikation mit dem Datenerfassungssubsystem, wobei das Prozessorsubsystem dazu ausgebildet ist, das erste Signal zu empfangen und das erste Signal zu verarbeiten, um ein erstes Parametersignal zu erhalten, das repräsentativ für einen physiologischen Parameter oder einen Performanceparameter eines Subjekts (100) ist, und das erste Parametersignal an eine Datenbank zu übermitteln, wobei die Datenbank dazu ausgebildet ist, das erste Parametersignal zu empfangen und zu speichern,
wobei die Datenbank dazu ausgebildet ist, ein zweites Parametersignal, das repräsentativ ist für einen physiologischen Parameter oder einen Performanceparameter eines zweiten Subjekts, zu empfangen und zu speichern;
wobei das Datenbanksystem ferner ein Steuerungsdatenverarbeitungssubsystem beinhaltet, das dazu ausgebildet ist, das erste Parametersignal mit dem zweiten Parametersignal zu vergleichen, um Ähnlichkeiten zwischen den Parameterinformationen, die in dem ersten Parametersignal und dem zweiten Parametersignal enthalten sind, festzustellen, und
wobei die Datenbank dazu ausgebildet ist, das erste Parametersignal in Zuordnung zu demographischen Informationen über das Subjekt (100) und historischen athletischen Performancedaten des Subjekts (100) zu speichern;
und **dadurch gekennzeichnet, dass**
das Steuerungsdatenverarbeitungssubsystem dazu ausgebildet ist, festzustellen, ob das erste Parametersignal und das zweite Parametersignal ein gemeinsames Attribut teilen, wobei das Attribut ein athletischer Performancelevel oder eine Sportart ist,
und wobei das Steuerungsdatenverarbeitungssubsystem dazu ausgebildet ist, eine Datenbankteilmenge beinhaltend das erste und zweite Parametersignal zu speichern, wenn festgestellt wird, dass das erste und zweite Parametersignal das Attribut teilen.

2. Datenbanksystem nach Anspruch 1, wobei Mustererkennungsalgorithmen angewendet werden, um festzustellen, ob ein Parametersignal zumindest eines repräsentiert aus: Performanceverschlechterung, Performanceverbesserung, und Ansprechen auf Training.

3. Datenbanksystem nach Anspruch 1, wobei das Datenerfassungssubsystem einen Sensor (24) beinhaltet, welcher nahe dem Brustbereich des Subjekts positioniert ist.

4. Datenbanksystem nach Anspruch 3, wobei der Sensor (24) ein Paar elektromagnetischer Spulen (24a, 24b) beinhaltet, welche auf Abstandsänderungen zwischen ihnen ansprechen.

5. Datenbanksystem nach Anspruch 4, wobei das Signal einen respiratorischen Parameter repräsentiert, welcher durch eine Abstandsänderung zwischen dem Paar elektromagnetischer Spulen (24a, 24b) angezeigt wird.

6. Verfahren zum Verarbeiten athletischer Performancedaten, das Verfahren beinhaltend:
Empfangen eines ersten Signals von einem ersten Subjekt (100);
Verarbeiten des ersten Signals mit einem Prozessorsubsystem, um ein erstes Parametersignal zu erhalten, das repräsentativ ist für einen physiologischen Parameter oder einen Performanceparameter des ersten Subjekts (100),
Übermitteln des ersten Parametersignals an eine Datenbank;
Zuordnen des ersten Parametersignals zu demographischen Informationen und einer Performancehistorie des ersten Subjekts (100);
Speichern des ersten Parametersignals in der Datenbank in Zuordnung zu den demographischen Informationen über das erste Subjekt (100) und der Performancehistorie des ersten Subjekts (100);
Vergleichen des ersten Signals mit einem zweiten Parametersignal in der Datenbank, wobei das zweite Parametersignal einen physiologischen Parameter oder einen Performanceparameter eines zweiten Subjekts repräsentiert;
und **gekennzeichnet durch**
Feststellen eines gemeinsamen Attributs bei dem ersten und zweiten Subjekt basierend auf dem ersten und zweiten Parametersignal, wobei das Attribut ein athletischer Performancelevel oder eine Sportart ist; und
Speichern einer Datenbankteilmenge, wenn festgestellt wird, das ein Attribut geteilt wird, wobei die Datenbankteilmenge das erste und zweite Parametersignal beinhaltet.

7. Verfahren nach Anspruch 6, weiter beinhaltend ein Anwenden von Mustererkennungsalgorithmen, um festzustellen, ob das erste Parametersignal eine Performanceänderung anzeigt verglichen mit einem früher gespeicherten Parametersignal von dem ersten Subjekt.

## Revendications

1. Un système de base de données pour le traitement de données de performances athlétiques, le système de base de données comprenant :
un sous-système d'acquisition de données configuré pour générer et émettre un premier signal ; et
un sous-système processeur en communication avec le sous-système d'acquisition de données, le sous-système processeur étant configuré pour recevoir le premier signal et traiter le premier signal pour obtenir un premier signal de paramètre qui est représentatif d'un paramètre physiologique ou d'un paramètre de performance d'un patient (100) et pour émettre le premier signal de paramètre vers une base de données, la base de données étant configurée pour recevoir et stocker le premier signal de paramètre,
dans lequel la base de données est configurée pour recevoir et stocker un second signal de paramètre représentatif d'un paramètre physiologique ou d'un paramètre de performance d'un second patient ;
dans lequel le système de base de données comprend en outre un sous-système de traitement de données de contrôle configuré pour comparer le premier signal de paramètre au second signal de paramètre pour déterminer des similarités entre les informations de paramètre contenues dans le premier signal de paramètre et dans le second signal de paramètre, et
dans lequel la base de données est configurée pour stocker le premier signal de paramètre en association avec des informations démographiques du patient (100) et des données d'historique de performances athlétiques du patient (100) ;
et **caractérisé en ce que**
le sous-système de traitement de données de contrôle est configuré pour déterminer si le premier signal de paramètre et le second signal de paramètre partagent un attribut commun, l'attribut étant un niveau de performance athlétique ou un type de sport,
et dans lequel, s'il est déterminé que le premier et le second signal de paramètre partagent l'attribut, le sous-système de traitement de données de contrôle est configuré pour stocker un sous-ensemble de base de données qui comprend le premier et le second signal de paramètre.

2. Le système de base de données de la revendication 1, dans lequel des algorithmes de reconnaissance de forme sont appliqués pour déterminer si un signal de paramètre représente au moins l'une d'entre une dégradation des performances, une amélioration des performances et une réponse à un entrainement.

3. Le système de base de données de la revendication 1, dans lequel le sous-système d'acquisition de données comprend en outre un capteur (24) positionné à proximité de la région thoracique du patient.

4. Le système de base de données de la revendication 3, dans lequel le capteur (24) comprend une paire de bobines électromagnétiques (24a, 24b) sensibles à des modifications d'une distance qui les sépare.

5. Le système de base de données de la revendication 4, dans lequel les signaux représentent un paramètre respiratoire indiqué par une modification de la distance entre la paire de bobines électromagnétiques (24a, 24b).

6. Un procédé de traitement de données de performances athlétiques, le procédé comprenant :
la réception d'un premier signal en provenance d'un premier patient (100) ;
le traitement du premier signal par un sous-système processeur pour obtenir un premier signal de paramètre qui est représentatif d'un paramètre physiologique ou d'un paramètre de performance du premier patient (100) ;
l'émission du premier signal de paramètre vers une base de données ;
l'association du premier signal de paramètre à des informations démographiques et à un historique de performances du premier patient (100) ;
le stockage du premier signal de paramètre dans la base de données en association avec les informations démographiques du premier patient (100) et l'historique de performances du premier patient (100) ;
la comparaison du premier signal à un second signal de paramètre de la base de données, ce second signal de paramètre représentant un paramètre physiologique ou un paramètre de performance d'un second patient ;
et **caractérisé par**
la détermination d'un attribut commun entre le premier et le second sujet sur la base du premier et du second signal de paramètre, l'attribut étant un niveau de performance athlétique ou un type de sport ; et
le stockage d'un sous-ensemble de base de données s'il est déterminé qu'un attribut est partagé, le sous-ensemble de base de données comprenant le premier et le second signal de paramètre.

7. Le procédé de la revendication 6, comprenant en outre l'application d'algorithmes de reconnaissance de forme pour déterminer si le premier signal de paramètre indique une modification des performances par rapport à un signal de paramètre précédemment mémorisé venant du premier patient.
